# EUROPEAN PATENT APPLICATION

(11) **EP 2 006 299 A1**
(43) Date of publication of application: **24.12.2008**
(21) Application number: 07301124.9
(22) Date of filing: 18.06.2007
(51) Int. Cl.: C07K 14/705

(54) **Identification of new isoforms of the MHC-class I specific receptor CD160 and uses thereof**

(71) Applicant: INSTITUT NATIONAL DE LA SANTE ET DE LA RECHERCHE MEDICALE (INSERM), 75013 Paris (FR)
(72) Inventor: Bensussan, Armand, 75013 Paris (FR); Marie-Cardine, Anne, 94000 Créteil (FR)
(74) Representative: Ahner, Francis

(57) **Abstract**

The present invention concerns an isolated polypeptide comprising a sequence selected in the group comprising SEQ ID NO.1, SEQ ID NO.3, their orthologs, and derivatives thereof; a polynucleotide comprising a nucleic acid sequence encoding for said polypeptide; a vector comprising said polynucleotide; a host cell genetically engineered with said polynucleotide or with said vector; a pharmaceutical composition comprising said polypeptide, a polynucleotide coding for said polypeptide or a vector comprising said polynucleotide, and optionally a pharmaceutically acceptable carrier; a method for screening for antagonists and/or agonists of said polypeptide; and uses of said polypeptide, a polynucleotide coding for said polypeptide or a vector comprising said polynucleotide, for the manufacture of a medicament.

SEQUENCE LISTING

<110> INSTITUT NATIONAL DE LA SANTE ET DE LA RECHERCHE MEDICALE
      (INSERM)
      BENSUSSAN, Armand
      CARDINE, Anne-Marie

<120> Identification of new isoforms of the MHC-class I specific
      receptor CD160 and uses thereof

<130> 66892/D25335

<160> 21

<170> PatentIn version 3.3


<210> 1
<211> 234
<212> PRT
<213> Homo sapiens

<400> 1
Met Leu Leu Glu Pro Gly Arg Gly Cys Cys Ala Leu Ala Ile Leu Leu
1               5                   10                  15
Ala Ile Val Asp Ile Gln Ser Gly Gly Cys Ile Asn Ile Thr Ser Ser
            20                  25                  30
Ala Ser Gln Glu Gly Thr Arg Leu Asn Leu Ile Cys Thr Val Trp His
        35                  40                  45
Lys Lys Glu Glu Ala Glu Gly Phe Val Val Phe Leu Cys Lys Asp Arg
    50                  55                  60
Ser Gly Asp Cys Ser Pro Glu Thr Ser Leu Lys Gln Leu Arg Leu Lys
65                  70                  75                  80
Arg Asp Pro Gly Ile Asp Gly Val Gly Glu Ile Ser Ser Gln Leu Met
                85                  90                  95
Phe Thr Ile Ser Gln Val Thr Pro Leu His Ser Gly Thr Tyr Gln Cys
            100                 105                 110
Cys Ala Arg Ser Gln Lys Ser Gly Ile Arg Leu Gln Gly His Phe Phe
        115                 120                 125
Ser Ile Leu Phe Thr Glu Thr Gly Asn Tyr Thr Val Thr Gly Leu Lys
    130                 135                 140
Gln Arg Gln His Leu Glu Phe Ser His Asn Glu Gly Thr Leu Ser Ser
145                 150                 155                 160
Gly Phe Leu Gln Glu Lys Val Trp Val Met Leu Val Thr Ser Leu Val
                165                 170                 175
Ala Leu Gln Gly Met Ser Lys Arg Ala Val Ser Thr Pro Ser Asn Glu
            180                 185                 190
Gly Ala Ile Ile Phe Leu Pro Pro Trp Leu Phe Ser Arg Arg Arg Arg
        195                 200                 205
Leu Glu Arg Met Ser Arg Gly Arg Glu Lys Cys Tyr Ser Ser Pro Gly
    210                 215                 220
Tyr Pro Gln Glu Ser Ser Asn Gln Phe His
225                 230


<210> 2
<211> 72
<212> PRT
<213> Homo sapiens

<400> 2

Met Leu Leu Glu Pro Gly Arg Gly Cys Cys Ala Leu Ala Ile Leu Leu
1               5                   10                  15
Ala Ile Val Asp Ile Gln Ser Gly Glu Thr Gly Asn Tyr Thr Val Thr
            20                  25                  30
Gly Leu Lys Gln Arg Gln His Leu Glu Phe Ser His Asn Glu Gly Thr
        35                  40                  45
Leu Ser Ser Gly Phe Leu Gln Glu Lys Val Trp Val Met Leu Val Thr
    50                  55                  60
Ser Leu Val Ala Leu Gln Ala Leu
65                  70


<210> 3
<211> 27
<212> PRT
<213> Homo sapiens

<400> 3
Glu Thr Gly Asn Tyr Thr Val Thr Gly Leu Lys Gln Arg Gln His Leu
1               5                   10                  15
Glu Phe Ser His Asn Glu Gly Thr Leu Ser Ser
            20                  25


<210> 4
<211> 24
<212> PRT
<213> Artificial sequence

<220>
<223> Signal peptide

<400> 4
Met Leu Leu Glu Pro Gly Arg Gly Cys Cys Ala Leu Ala Ile Leu Leu
1               5                   10                  15
Ala Ile Val Asp Ile Gln Ser Gly
            20


<210> 5
<211> 21
<212> PRT
<213> Artificial sequence

<220>
<223> GPI anchor domain

<400> 5
Gly Phe Leu Gln Glu Lys Val Trp Val Met Leu Val Thr Ser Leu Val
1               5                   10                  15
Ala Leu Gln Ala Leu
            20


<210> 6
<211> 109
<212> PRT
<213> Artificial sequence

<220>
<223> Ig domain

<400> 6
Gly Cys Ile Asn Ile Thr Ser Ser Ala Ser Gln Glu Gly Thr Arg Leu
1               5                   10                  15
Asn Leu Ile Cys Thr Val Trp His Lys Lys Glu Glu Ala Glu Gly Phe
            20                  25                  30
Val Val Phe Leu Cys Lys Asp Arg Ser Gly Asp Cys Ser Pro Glu Thr
        35                  40                  45
Ser Leu Lys Gln Leu Arg Leu Lys Arg Asp Pro Gly Ile Asp Gly Val
    50                  55                  60
Gly Glu Ile Ser Ser Gln Leu Met Phe Thr Ile Ser Gln Val Thr Pro
65                  70                  75                  80
Leu His Ser Gly Thr Tyr Gln Cys Cys Ala Arg Ser Gln Lys Ser Gly
                85                  90                  95
Ile Arg Leu Gln Gly His Phe Phe Ser Ile Leu Phe Thr
            100                 105


<210> 7
<211> 55
<212> PRT
<213> Artificial sequence

<220>
<223> Intracellular membrane

<400> 7
Gly Met Ser Lys Arg Ala Val Ser Thr Pro Ser Asn Glu Gly Ala Ile
1               5                   10                  15
Ile Phe Leu Pro Pro Trp Leu Phe Ser Arg Arg Arg Arg Leu Glu Arg
            20                  25                  30
Met Ser Arg Gly Arg Glu Lys Cys Tyr Ser Ser Pro Gly Tyr Pro Gln
        35                  40                  45
Glu Ser Ser Asn Gln Phe His
    50                  55


<210> 8
<211> 625
<212> DNA
<213> Homo sapiens

<400> 8
atgctgttgg aacccggcag aggctgctgt gccctggcca tcctgctggc aattgtggac      60
atccagtctg gtggatgcat taacatcacc agctcagctt cccaggaagg aacgcgacta     120
aacttaatct gtactgtatg gcataagaaa gaagaggctg aggggtttgt agtgtttttg     180
tgcaaggaca ggtctggaga ctgttctcct gagaccagtt taaaacagct gagacttaaa     240
agggatcctg ggatagatgg tgttggtgaa atatcatctc agttgatgtt caccataagc     300
caagtcacac cgttgcacag agacagggaa ctacacagtg acgggattga aacaaagaca     360
acaccttgag ttcagccata atgaaggcac tctcagttca ggcttcctac aagaaaaggt     420
ctgggtaatg ctggtcacca gccttgtggc ccttcaaggt atgtccaaaa gagccgtaag     480
caccccaagc aatgagggtg ctattatatt tctgccacct tggttattct ccaggaggag     540
aaggttggaa aggatgtcca gggggagaga aaaatgttac tcaagccctg gctatccaca     600
ggaaagttca aaccagtttc actga                                           625


<210> 9
<211> 219
<212> DNA
<213> Homo sapiens

<400> 9
atgctgttgg aacccggcag aggctgctgt gccctggcca tcctgctggc aattgtggac      60
atccagtctg gtgagacagg gaactacaca gtgacgggat tgaaacaaag acaacacctt     120
gagttcagcc ataatgaagg cactctcagt tcaggcttcc tacaagaaaa ggtctgggta     180
atgctggtca ccagccttgt ggcccttcaa gctttgtaa                            219

<210> 10
<211> 22
<212> DNA
<213> Artificial sequence

<220>
<223> Primer

<400> 10
atgctgttgg aacccggcag ag                                               22

<210> 11
<211> 21
<212> DNA
<213> Artificial sequence

<220>
<223> Primer

<400> 11
ttacaaagct tgaagggcca c                                                21

<210> 12
<211> 27
<212> DNA
<213> Artificial sequence

<220>
<223> Primer

<400> 12
tcagtgaaac tggtttgaac tttcctg                                          27

<210> 13
<211> 24
<212> DNA
<213> Artificial sequence

<220>
<223> Primer

<400> 13
atggccttac cagtgaccgc cttg                                             24

<210> 14
<211> 47
<212> DNA
<213> Artificial sequence

<220>
<223> Primer

<400> 14
ggggtgctta cggctctttt ggagttgcag taaagggtga taaccag                47

<210> 15
<211> 47
<212> DNA
<213> Artificial sequence

<220>
<223> Primer

<400> 15
ctggttatca ccctttactg caactccaaa agagccgtaa gcacccc                47

<210> 16
<211> 14
<212> PRT
<213> Artificial sequence

<220>
<223> Polypeptide

<400> 16

Ser Ser Ala Ser Gln Glu Gly Thr Arg Leu Asn Leu Ile Cys
1               5                   10

<210> 17
<211> 16
<212> PRT
<213> Artificial sequence

<220>
<223> Polypeptide

<400> 17

Lys Gln Arg Gln His Leu Glu Phe Ser His Asn Asn Glu Gly Thr Leu
1               5                   10                  15

<210> 18
<211> 3389
<212> DNA
<213> Homo sapiens

<400> 18
aacaagatgc aaggacaatc tgaggactga gagccatttc aacgtgagcc cccagtctga      60
gaacaagaaa gaagaacttc tgtctcgaga ctgaagccaa ggataccagc agagccaaca     120
tttgcttcaa gttcctgggc ctgctgacag cgtgcaggat gctgttggaa cccggcagag     180
gctgctgtgc cctggccatc ctgctggcaa ttgtggacat ccagtctggt gagacaggga     240
actacacagt gacgggattg aaacaaagac aacaccttga gttcagccat aatgaaggca     300
ctctcagttc aggcttccta caagaaaagg tctgggtaat gctggtcacc agccttgtgg     360
cccttcaagg tatgtccaaa agagccgtaa gcaccccaag caatgagggt gctattatat     420
ttctgccacc ttggttattc tccaggagga gaaggttgga aaggatgtcc agggggagag     480
aaaaatgtta ctcaagccct ggctatccac aggaaagttc aaaccagttt cactgatcca     540
aaggcactaa ggaggaagac agataaatca gacttttcaa tgggtaggag gtaagactca     600

ttaggaatga aggcctgtga gagaggcaag ttagtacaac tacctctact cactgcatga 660
acagagggct acaagttgaa gcaacagatt ttcagctatt agaggaagaa aaggcaagag 720
acagacttgg tttggagatg ggttttgaac aatgcggctc ctcagcaaag gctgatggcc 780
agattgagta tattaaacac atgtagctga gtcctctgaa aaccatttac caatatagat 840
tagcgttttc cctattagat aacaaaaagt gaaacttcca ataatttgtc ataacgtttt 900
tgcataacaa gcaactgatt caggtttttg cctggccttg gcattcttaa ctccatttta 960
tttaggcatt cctcaaatca ataactacca ctgttttgaa tggcatcaga atgtaagctc 1020
tatatatatg taaggacttt gtcttcttta ccccaacgaa aagtcccaca catatggcag 1080
gctcttaata aatttgttaa aaaacctgta tgatgaatca gatgctttac acatgatgat 1140
tctaacctta tacaaccctt gcaagcaaac atcccctggt tactcatcag gaaacagaca 1200
caaggaagtt aaatagggcc tggtgcggtg gctcacgcct gtaatcccag cactttggga 1260
ggccaaggca ggcagatcct ttgagcctag gagtttgagg ccagtctggg caacatggca 1320
aaaccccaac tatacaaaaa aatacaaaaa ttaggcatgg tggcacacaa ctgtagtccc 1380
aagctaaggc atgaggatca cctgagccca ggctggttga ggttgcagtg agccatgatc 1440
atgccactgt actccagcct gggtgacagg gtgagacact ttctcaaagg aaaaaaaaaa 1500
aaaaaagagt taattgttgc ccgtcacagc aattaataca gatccttctc actctaaaac 1560
cttacactca gtaatgatct actatatgca aagcacctct tacatccttc accaccccat 1620
tcattctgag attgactcct cccctttcca gaaacaggac cacctaaaca ttccctattc 1680
cagtccctta tttgcaagtc atcaaaagaa aacagagatt tcaaaaagaa aacagagacc 1740
ttatctgtaa tccttggtaa ggatcttcca aatttaagcc tatgccagtt ttcaggagga 1800
aacatgaata tgaaacagtc tgacctttag acataatttt ttgttgactt taaattccct 1860
tggttaggcc actgtaacca gtaaaataaa ttttatttta gaataaaaat tataaagggg 1920
aaattgagtc tagccataca acctactact agttactatc tttgattccc aagtcatcct 1980
agagcaaaaa aaaaaaaaaa aaaaaatcat ctctggcctt tttttccttt tccactcagg 2040
tgaagagaat gagcttttga atccaatttt ttgttttctc ctatattcaa ttcaagtagc 2100
tttaaagtta accaaaggaa aaaaccaaag ctcaatattt gattaaacga gaaaatatac 2160
actacttaga agctttcttt tctttatcct tcttccccaa ctcaaggctt agtagaatta 2220
ttgaaagagg tagtattcag tagaatagta cacatgcttt ttcaggtcca gaaacaccac 2280
ataggtcaag ttatcatgct tacatgtggg gaaaaggagg agggagtttc ctggacctct 2340
gtgcctctct cagctcatga actagtttca gtgaatatcc ttgtcttacc actgtgccaa 2400
caattcagag ctatacgtgg aaaacctacc ttcaactttt tgagtggcat ctattgcttc 2460
tttgattgtt ccttccagca aggagcgatc aaaggctagc cccttcccca ctcccaccag 2520
aatcagattt gctcaagatt acaaccctaa tttagtaata agatgcaata cataaaatag 2580
ttcccacttt ccttttccaa tccctttaca gtcatgaggg cctatcacgc acgcatctca 2640
ggacaggtga gacttcatta tatcaggtat tttgttgcta ttctaagtta taaggcagta 2700
atacaaactt tctttccaca gctttgtaag ccttgtgcca aaagaaactt ttaaaacagc 2760
tacagcaaga tgagtctgac tatggcttag tatctttctc attacaatag gcacagagaa 2820
gaatgcaaca gggcacaggg gaagagatgc taaatatacc aagaatctgt ggaaatataa 2880
gctggggcaa atcagtgtaa tccttgactt tgctcctcac catcagggca aacttgcctt 2940
cttccctcct aagctccagt aaataaacag aacagctttc accaaagtgg gtagtatagt 3000
cctcaaatat cggataaata tatgcgtttt tgtaccccag aaaaactttt cctccctctt 3060
catcaacata gtaaaataag tcaaacaaaa tgagaacacc aaattttggg ggaataaatt 3120
tttatttaac actgcaaagg aaagagagag aaaacaagca aagataggta ggacagaaag 3180
gaagacagcc agatccagtg attgacttgg catgaaaatg agaaaatgca gacagacctc 3240
aacattcaac aacatccata cagcactgct ggaggaagag gaagatttgt gcagaccaag 3300
agcaccacag actacaactg cccagcttca tctaaatact tgttaacctc tttggtcatt 3360
tctcttttta aataaatgcc catagcagt 3389

<210> 19
<211> 125
<212> PRT
<213> Homo sapiens

<400> 19
Met Leu Leu Glu Pro Gly Arg Gly Cys Cys Ala Leu Ala Ile Leu Leu
1               5                   10                  15
Ala Ile Val Asp Ile Gln Ser Gly Glu Thr Gly Asn Tyr Thr Val Thr
            20                  25                  30
Gly Leu Lys Gln Arg Gln His Leu Glu Phe Ser His Asn Glu Gly Thr
        35                  40                  45

Leu Ser Ser Gly Phe Leu Gln Glu Lys Val Trp Val Met Leu Val Thr
    50                  55                  60
Ser Leu Val Ala Leu Gln Gly Met Ser Lys Arg Ala Val Ser Thr Pro
65                  70                  75                  80
Ser Asn Glu Gly Ala Ile Ile Phe Leu Pro Pro Trp Leu Phe Ser Arg
                85                  90                  95
Arg Arg Arg Leu Glu Arg Met Ser Arg Gly Arg Glu Lys Cys Tyr Ser
            100                 105                 110
Ser Pro Gly Tyr Pro Gln Glu Ser Ser Asn Gln Phe His
        115                 120                 125

<210> 20
<211> 58
<212> PRT
<213> Artificial sequence

<220>
<223> sCD160 delta Ig-GPI-Flag

<400> 20
Met Leu Leu Glu Pro Gly Arg Gly Cys Cys Ala Leu Ala Ile Leu Leu
1               5                   10                  15
Ala Ile Val Asp Ile Gln Ser Gly Glu Thr Gly Asn Tyr Thr Val Thr
            20                  25                  30
Gly Leu Lys Gln Arg Gln His Leu Glu Phe Ser His Asn Glu Gly Thr
        35                  40                  45
Leu Ser Ser Asp Tyr Lys Asp Asp Asp Lys
    50                  55

<210> 21
<211> 167
<212> PRT
<213> Artificial sequence

<220>
<223> sCD160-Flag

<400> 21
Met Leu Leu Glu Pro Gly Arg Gly Cys Cys Ala Leu Ala Ile Leu Leu
1               5                   10                  15
Ala Ile Val Asp Ile Gln Ser Gly Gly Cys Ile Asn Ile Thr Ser Ser
            20                  25                  30
Ala Ser Gln Glu Gly Thr Arg Leu Asn Leu Ile Cys Thr Val Trp His
        35                  40                  45
Lys Lys Glu Glu Ala Glu Gly Phe Val Val Phe Leu Cys Lys Asp Arg
    50                  55                  60
Ser Gly Asp Cys Ser Pro Glu Thr Ser Leu Lys Gln Leu Arg Leu Lys
65                  70                  75                  80
Arg Asp Pro Gly Ile Asp Gly Val Gly Glu Ile Ser Ser Gln Leu Met
                85                  90                  95
Phe Thr Ile Ser Gln Val Thr Pro Leu His Ser Gly Thr Tyr Gln Cys
            100                 105                 110
Cys Ala Arg Ser Gln Lys Ser Gly Ile Arg Leu Gln Gly His Phe Phe
        115                 120                 125
Ser Ile Leu Phe Thr Glu Thr Gly Asn Tyr Thr Val Thr Gly Leu Lys
    130                 135                 140
Gln Arg Gln His Leu Glu Phe Ser His Asn Glu Gly Thr Leu Ser Ser
145                 150                 155                 160
Asp Tyr Lys Asp Asp Asp Lys

                165

## Description

### Background of the Invention

### Field of the invention

The invention relates to the field of immunology, and in particular to new CD160 isoforms and uses thereof.

### Description of prior art

Natural killer (NK) lymphocytes recognize abnormal or aberrant cells through multiple receptors that detect normal host molecules, as well as stress-induced or pathogen-expressed motifs (RAULET, Nat. Immunol., vol.5, p:996-1002, 2004; LANIER, Annu. Rev. Immunol., vol.23, p:225-74, 2005). Individual NK cells express both activating and inhibitory receptors, which together drive the specificity towards target cells (LANIER, 2005, abovementioned). The NK cell inhibitory receptors have been classified into three groups, namely the heterodimeric CD94/NKG2A, the Ig-like transcript (ILT) receptors, and the members of the killer cell Ig-like receptors (KIRs; PARHAM, Nat. Rev. Immunol., vol.5, p:201-14, 2005; NATARAJAN et al., Annu. Rev. Immunol., vol.20, p:853-85, 2002). All of them bind to classical or non classical MHC class I molecules.

A common characteristic of the inhibitory receptors is the presence of one or more immunoreceptor tyrosine-based inhibition motifs (ITIM) within their intracellular tail (BORREGO et al., Mol. Immunol., vol.38, p:637-60, 2002). Following engagement by their ligands, the inhibitory receptors become phosphorylated on the tyrosine residue(s) present in the ITIM(s), creating docking sites for the SH2 domains of the cytoplasmic protein tyrosine phosphatases SHP1 and SHP2. The recruitment of the phosphatase further results in the down-regulation of the intracellular activation cascade (YUSA et al., J. Immunol., vol.168, p:5047-5057, 2002).

In contrast, the activating receptors recognize a large variety of ligands, and present more complex but well characterized signaling pathways. Natural cytotoxicity receptors (NCRs) and NKG2D are the major receptors involved in NK cytotoxicity (BOTTINO et al., Hum. Immunol., vol.61, p:1-6, 2000). The NCRs (namely NKp46, NKp44 and NKp30) belong to the Ig-superfamily, and represent non-MHC class I specific receptors whose cellular ligands remained to be identified. These activating receptors transduce signals through their association with ITAM-containing molecules such as CD3ζ, FcεRIγ and DAP12 (VIVIER et al., Science, vol. 306, p:1517-1519, 2004; MORETTA et al., Immunol. Today, vol.21, p:228-234, 2000). Besides the NCRs, NKG2D is C-type lectin-like receptor shown to recognize the MHC-class I homologues MICA and MICB, and the family of UL16-binding proteins (ULBP1-4; GONZALEZ et al., Curr. Top. Microbiol. Immunol., vol.298, p:121-123, 2006). Unlike the NCRs, NKG2D uses the transmembrane polypeptide DAP10 for signaling, which interacts with the PI3-kinase once phosphorylated (CERWENKA & LANIER, Immunol. Rev., vo1.181, p:158-169, 2001). More recently the characterization of NKp80, an activating receptor exclusively expressed by human NK cells, has been reported (VITALE et al., Eur. J. Immunol., vol.31, p:233-242, 2001). A search for NKp80 ligands led to the identification of activation-induced C-type lectin (AICL; WELTE et al., Nat. Immunol., vol.7, p:1334-1342, 2006). However, NKp80 signaling pathway remains enigmatic as this receptor does not contain a transmembrane charged residue (a feature allowing association with ITAM-containing adaptor proteins) or any intracellular consensus activation motifs (VITALE *et al.,* 2001, abovementioned). Finally, among the activating receptors, the role of CD94/NKG2C, and of DAP12-associated KIRs, has also been well defined (OLCESE et al., J. Immunol., vol.158, p:5083-5086, 1997; LANIER et al., Immunity, vol.8, p:693-701, 1998).

More recently the identification of the MHC class I specific receptor BY55/CD160 has been reported (ANUMANTHAN et al., J. Immunol., vol.161, p:2780-2790, 1998). CD160 appears to be unique among the activating receptors since *CD160* gene was found to be located on human chromosome 1, and the corresponding protein was characterized as a glycosylphosphatidylinositol (GPI)-anchored cell surface molecule. CD160 is expressed by intestinal intraepithelial T lymphocytes and by a minor subset of circulating lymphocytes including NK cells, TCRγδ and cytotoxic effector CD8^{bright}CD28⁻ T lymphocytes (ANUMANTHAN *et al.,* 1998, abovementioned; MAIZA et al., J. Exp. Med., vol.178, p:1121-1126, 1993). Furthermore, it shows a broad specificity for the MHC class Ia and Ib molecules. We initially demonstrated that CD160 exerts a co-receptor function on CD3-activated-CD4⁺ T cell clones (AGRAWAL et al., J. Immunol., vol.162, p:1223-1226, 1999) and CD8^{bright} CD28⁻ cytotoxic effector T lymphocytes (NIKOLOVA et al., Int. Immunol., vol. 14, p:445-451, 2002). In addition, CD160 exhibits a full activatory receptor function on NK lymphocytes, as demonstrated by the induction of their cytotoxic potential upon interaction with HLA-C molecules (LE BOUTEILLER et al., Proc. Natl. Acad. Sci. U S A, vol.99, p:16963-16968, 2002). The engagement of CD160 by its physiological ligand also results in a unique profile of cytokine secretion by cytotoxic NK cells, with the release of TNFα, IFNγ and IL-6 (BARAKONYI et al., J. Immunol., vo1.173, p:5349-5354, 2004). Thus CD160 might be involved in mechanisms regulating both adaptive and innate immunity. Finally, we recently reported that a down-modulation of CD160 surface expression occurs as a consequence of its proteolytic cleavage upon NK cell activation (GIUSTINIANI et al., J. Immunol., vol. 178, p:1293-1300, 2007). The released soluble form of CD160 was found to impair the MHC class I-specific cytotoxicity of CD8⁺ T lymphocytes and NK cells.

### Summary of the invention

In accordance with the present invention there is provided an isolated polypeptide comprising a sequence selected in the group comprising SEQ ID NO.1, SEQ ID NO.3, their orthologs, and derivatives thereof.

In accordance with the present invention there is also provided a polynucleotide comprising a nucleic acid sequence encoding for said polypeptide, a vector comprising said polynucleotide, a host cell genetically engineered with said polynucleotide or with said vector.

In accordance with the present invention, there is also provided a pharmaceutical composition comprising a polypeptide comprising a sequence selected in the group comprising SEQ ID NO.3, its orthologs and derivatives thereof, a polynucleotide coding for said polypeptide or a vector comprising said polynucleotide, and optionally a pharmaceutically acceptable carrier.

In accordance with the present invention, there is also provided a method for screening for antagonists and/or agonists of the polypeptide as defined previously comprising:
a) expressing the receptor corresponding to the polypeptide comprising a sequence selected in the group comprising SEQ ID NO.1, its orthologs and derivatives thereof on the surface of a cell;
b) contacting the cell with a receptor ligand and a compound to be screened;
c) determining whether a second signal is generated from the interaction of the ligand and the receptor; and
d) identifying if the compound to be screened is an agonist or an antagonist.

In accordance with the present invention, there is also provided the use of a polypeptide comprising a sequence selected in the group comprising SEQ ID NO.3, its orthologs, and derivatives thereof, a polynucleotide coding for said polypeptide or a vector comprising said polynucleotide, for the manufacture of a medicament for treating and/or preventing inflammatory diseases such as rheumatoid arthritis, atopic dermatitis, psoriasis, multiple sclerosis, diabetes, lupus and inflammatory bowel diseases, inflammatory conditions such as tissue graft or organ rejection, or autoimmune diseases in a subject.

In accordance with the present invention, there is finally provided the use of a polypeptide comprising a sequence selected in the group comprising SEQ ID NO.3, its orthologs, and derivatives thereof, a polynucleotide coding for said polypeptide or a vector comprising said polynucleotide, for the manufacture of a medicament for treating and/or preventing bacterial, viral, fungal or parasitic infection and/or for treating and/or preventing cancer.

### Brief Description of the Drawings

The figure 1 shows the identification of novel CD160-related coding sequences.
The figure 2 shows sequence and molecular organisation of CD160 isoforms.
The figure 3 shows CD160 isoforms mRNA synthesis upon IL-15 activation of PK-NB cells.
The figure 4 shows the switch in CD160 isoforms expression following PK-NB cell activation.
The figure 5 shows the molecular characterization of CD160-TM.
The figure 6 shows the functional characterization of CD160-TM.

### Description of the Preferred embodiments

The inventors have now identified and characterised new isoforms of Homo sapiens CD160 and established the functions of said isoforms in activation of PB-NK lymphocytes.

Consequently, a first object of the present invention relates to a polypeptide comprising a sequence selected in the group comprising SEQ ID NO.1, SEQ ID NO.3, their orthologs, and derivatives thereof.

As used herein, the term "orthologs" refers to proteins in different species than the proteins SEQ ID NO.1 and SEQ ID NO.3 in *Homo sapiens* that evolved from a common ancestral gene by speciation. One of skill in the art in view of the specification and of its general knowledge can simply identify such orthologs.

As used herein, the term "derivatives"' refer to a polypeptide having a percentage of identity of at least 80% with complete SEQ ID NO.1, complete SEQ ID NO.3 or orthologs thereof, preferably of at least 90%, as an example of at least 95%, and more preferably of at least 99%.

As used herein, "percentage of identity" between two amino acids sequences, means the percentage of identical amino-acids, between the two sequences to be compared, obtained with the best alignment of said sequences, this percentage being purely statistical and the differences between these two sequences being randomly spread over the amino acids sequences. As used herein, "best alignment" or "optimal alignment", means the alignment for which the determined percentage of identity (see below) is the highest. Sequences comparison between two amino acids sequences are usually realized by comparing these sequences that have been previously align according to the best alignment; this comparison is realized on segments of comparison in order to identify and compared the local regions of similarity. The best sequences alignment to perform comparison can be realized, beside by a manual way, by using the global homology algorithm developped by SMITH and WATERMAN (Ad. App. Math., vol.2, p:482, 1981), by using the local homology algorithm developped by NEDDLEMAN and WUNSCH (J. Mol. Biol., vol.48, p:443, 1970), by using the method of similarities developped by PEARSON and LIPMAN (Proc. Natl. Acd. Sci. USA, vol.85, p:2444, 1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA), by using the MUSCLE multiple alignment algorithms (Edgar, Robert C., Nucleic Acids Research, vol. 32, p:1792, 2004 ). To get the best local alignment, one can preferably used BLAST software, with the BLOSUM 62 matrix, or the PAM 30 matrix. The identity percentage between two sequences of amino acids is determined by comparing these two sequences optimally aligned, the amino acids sequences being able to comprise additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.

According to a preferred embodiment, said polypeptide comprises a sequence selected in the group consisting of SEQ ID NO.1, its orthologs, and derivatives thereof.

According to another preferred embodiment, said polypeptide comprises a sequence selected in the group consisting of SEQ ID NO.3, its orthologs and derivatives thereof.

Preferably, said polypeptide comprises a signal peptide enabling the secretion of the polypeptide, which polypeptide can be expressed at the cell membrane surface or in the extracellular medium, and more preferably the sequence SEQ ID NO.4.

Still preferably, said polypeptide comprises a Glycosyl Phosphatidyl Inositol (GPI) anchor domain, and more preferably the sequence SEQ ID NO.5.

Said Glycosyl Phosphatidyl Inositol (GPI) anchor domain enables the anchorage of the polypeptide on the extracellular cell membrane, and is cleaved by a metalloprotease after activation of NK cells by IL-15. Said metalloprotease cleaved this domain between the phenylalanine and the leucine residues.

Preferably, said polypeptide does not comprise any Ig domain, and more preferably does not comprise the sequence SEQ ID NO.6.

Still preferably, said polypeptide does not comprise any intracellular domain, and more preferably does not comprise the sequence SEQ ID NO.7.

Advantageously, said polypeptide comprises a sequence selected in the group consisting of SEQ ID NO.2, its orthologs and derivatives thereof.

A second object of the invention is related to a polynucleotide comprising a nucleic acid sequence encoding for a polypeptide as defined previously.

The polynucleotide of the present invention may be in the form of RNA or in the form of DNA, preferably in the form of DNA.

The DNA may be double-stranded or single-stranded.

According to a preferred embodiment, the polynucleotide comprises a sequence selected in the group comprising SEQ ID NO:8 and SEQ ID NO:9.

The polynucleotide of the invention may include the coding sequence of the polypeptide defined previously, additional coding sequence such as leader sequence or a proprotein sequence, and/or additional non-coding sequence, such as introns or non-coding sequence 5' and/or 3' of the coding sequence of SEQ ID NO.1 or SEQ ID NO.2.

A third object of the present invention relates to vectors comprising the polynucleotide as defined previously.

Said vector can be a cloning or an expression vector, preferably an expression vector, and may be for example in the form of a plasmid, a viral particle, a phage, etc.

Such vectors may include bacterial plasmids, phage DNA, baculovirus, yeast plasmids, vectors derived from combinations of plasmids and phage DNA, viral DNA such as vaccinia, adenovirus, fowl pox virus, and pseudorabies. Large numbers of suitable vectors are known to those of skill in the art and are commercially available. The following vectors are provided by way of example. Bacterial: pQE70, pQE60, pQE-9 (QIAGEN), pbs, pD10, phagescript, psiX174, pbluescript SK, pbsks, pNH8A, pNH16a, pNH18A, pNH46A (STRATAGENE), ptrc99a, pKK223-3, pKK233-3, pDR540, pRIT5 (PHARMACIA). Eukaryotic: pWLNEO, pSV2CAT, pOG44, pXT1, pSG (STRATAGENE), pSVK3, pBPV, pMSG, pSVL (PHARMACIA). However, any other vector may be used as long as it is replicable and viable in the host.

The polynucleotide sequence, preferably the DNA sequence in the expression vector is operatively linked to an appropriate expression control sequence(s) (promoter) to direct mRNA synthesis. As representative examples of such promoters, one can mentioned prokaryotic or eukaryotic promoters such as CMV immediate early, HSV thymidine kinase, early and late SV40, LTRs from retrovirus, and mouse metallothionein-I. The expression vector also contains a ribosome binding site for translation initiation and a transcription vector. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in E. coli.

The vector of the invention containing the appropriate polynucleotide sequence as herein above described, as well as an appropriate promoter or control sequence, may be employed to transform an appropriate host to permit the host to express the polypeptide.

As an example, transcription of a DNA encoding for the polypeptide described previously by higher eukaryotes can be increased by inserting an enhancer sequence into the vector. Enhancer are cis-acting elements of DNA, usually about from 10 to 300 pb that act on a promoter to increase its transcription. Examples of enhancer include the SV40 enhancer, the CMV early promoter enhancer, and adenovirus enhancers.

A forth aspect of the invention relates to a host cell genetically engineered with the polynucleotide or with the vector described previously.

As used herein, the term "host cell genetically engineered" relates to host cells which have been transduced, transformed or transfected.

As representative examples of appropriate hosts, one can cites bacterial cells, such as E. coli, Streptomyces, Salmonella typhimurium, fungal cells such as yeast, insect cells such as Sf9, animal cells such as CHO or COS, plant cells, etc. The selection of an appropriate host is deemed to be within the scope of those skilled in the art from the teachings herein.

The introduction of the construct (i.e., polynucleotide or vector described previously) into the host cell can be effected by method well known from one of skill in the art such as calcium phosphate transfection, DEAE-Dextran mediated transfection, or electroporation.

A fifth aspect of the invention relates to pharmaceutical composition comprising a polypeptide comprising a sequence selected in the group comprising SEQ ID NO.3, its orthologs, and derivatives thereof, a polynucleotide coding for said polypeptide or a vector comprising said polynucleotide, and optionally a pharmaceutically acceptable carrier.

Preferably, said polypeptide is as defined previously.

According to a preferred embodiment, said pharmaceutical composition is suitable for treating and/or preventing inflammatory diseases such as rheumatoid arthritis, atopic dermatitis, psoriasis, multiple sclerosis, diabetes, lupus and inflammatory bowel diseases, inflammatory conditions such as tissue graft or organ rejection, or autoimmune diseases.

Said composition can further include at least one immunosuppressive agent for treating and/or preventing inflammatory diseases, inflammatory conditions, or autoimmune diseases.

Immunosuppressive agents are well known from one of skill in the art. As an example, one can cites cyclosporine A, FK506, rapamycin, steroids such as glucocorticoid, cytostatics such as methotrextate, azathioprine, and monoclonal antibodies such as OKT3 and anti-TNF. Other immunosuppressive agents are described in Physician's desk Reference 50^{th} Edition (1996).

According to another preferred embodiment, said pharmaceutical composition is suitable for treating and/or preventing bacterial, viral, fungal or parasitic infection.

According to still another preferred embodiment, said pharmaceutical composition is suitable for treating and/or preventing cancer.

The phrase "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin.

The composition may comprise one or more additives (e.g., stabilizers, preservatives). See, generally, Ullmann's Encyclopedia of Industrial Chemistry, 6th Ed. (various editors, 1989-1998, Marcel Dekker); and Pharmaceutical Dosage Forms and Drug Delivery Systems (ANSEL et al., 1994, WILLIAMS & WILKINS).

A sixth aspect of the invention relates to a method for screening for antagonists and/or agonists of the polypeptide comprising a sequence selected in the group consisting of SEQ ID NO.1, its orthologs, and derivatives thereof comprising:
a) expressing the receptor corresponding to the polypeptide as defined previously on the surface of a cell;
b) contacting the cell with a receptor ligand and a compound to be screened;
c) determining whether a second signal is generated from the interaction of the ligand and the receptor; and
d) identifying if the compound to be screened is an agonist or an antagonist.

In general, such screening procedures involve providing appropriate cells which express the polypeptide comprising a sequence selected in the group consisting of SEQ ID NO. 1, its orthologs and derivatives thereof on their surface. In particular, a polynucleotide encoding said polypeptide is employed to transfect cells to thereby express the receptor of the invention. Such transfection may be accomplished by procedures as hereinabove described.

One such screening procedure involves the use of peripheral blood NK cells which are transfected to constitutively express the receptor of the invention.

Thus, for example, such assay may be employed for screening for a receptor agonist by contacting the peripheral blood NK cells which encode the receptor of the invention with a receptor ligand such as the monoclonal anti-CD160 antibody CL1-R2 (the hybridoma producing said monoclonal antibody has CNCM deposit number I-3204 and is described in International application PCT WO 2006/015886) or the CD160 physiological ligand HLA-C as described in BARAKONYI et al. (J. Immunol., vol.173(9), p:5349-5354, 2004), and a compound to be screened. Inhibition of the signal generated by the ligand indicates that a compound is a potential antagonist for the receptor of the invention, i.e., inhibits activation of the receptor.

The screen may be employed for determining an agonist by contacting such cells with compounds to be screened and determining whether such compound generates a signal, i.e., activates the receptor.

The determination of an activation of the receptor can be assessed by determining the cell proliferation, preferably the NK cell proliferation; the identification of a cell proliferation activation corresponding to a receptor activation. Such proliferation can be determined by methods well known from one of skill in the art such as the monitoring of tritiated thymidine incorporation.

The determination of an activation of the receptor can also be assessed by determining the activation of the Erk signaling pathway; the identification of an activation of the Erk signaling pathway corresponding notably to the phosphorylation of Erk. Such activation of the Erk signaling pathway can be determined by methods well known from one of skill in the art such as by Western blot using an anti-phospho-Erk mAb.

Another method involves screening for antagonists by determining inhibition of binding of labeled ligand to cells which have the receptor on the surface thereof. Such a method involves transfecting a eukaryotic cells with DNA encoding the receptor of the invention such that the cell expresses the receptor on its surface and contacting the cell with a potential antagonist in the presence of a labeled form of a known ligand, such as CL1-R2 monoclonal antibody. The ligand can be labeled, e.g., by radioactivity. The amount of labeled ligand bound to the receptor is measured, e.g., by measuring radioactivity of the receptors. If the potential agonist binds to the receptor as determined by a reduction of labeled ligand which binds to the receptor, the binding of labeled ligand to the receptor is inhibited.

In general, antagonists for the receptor of the invention which are determined by screening procedures may be employed in a variety of therapeutic purposes as immunosuppressive agents. For example, such antagonists can be employed for treating and/or preventing inflammatory diseases, inflammatory conditions, or autoimmune diseases.

A potential antagonist includes a siRNA construct prepared through the use of siRNA technology. siRNA technology can be used to control gene expression of the receptor of the invention.

Potential antagonists also include a soluble form of the receptor of the invention, e.g., a fragment of the receptor, which binds to the ligand and prevents the ligand from interacting with the receptor of the invention. As an example of such a soluble form of the receptor of the invention, one can cite the soluble polypeptide described in GIUSTINIANI et al. (J. Immunol., vo1.178(3), p:1293-1300, 2007).

Agonists for the receptor of the invention are also useful for therapeutic purposes, such as for treating and/or preventing bacterial, viral, fungal or parasitic infection, or for treating and/or preventing cancer.

A seventh aspect of the invention includes the use of a polypeptide comprising a sequence selected in the group comprising SEQ ID NO.3, its orthologs and derivatives thereof, a polynucleotide coding for said polypeptide or a vector comprising said polynucleotide, for the manufacture of a medicament for treating and/or preventing inflammatory diseases such as rheumatoid arthritis, atopic dermatitis, psoriasis, multiple sclerosis, diabetes, lupus and inflammatory bowel diseases, inflammatory conditions such as tissue graft or organ rejection, or autoimmune diseases in a subject.

As used herein "Subject" means a mammal including without limitation humans, non-human primates, farm animals, domestic animals and laboratory animals, preferably said subject is a human.

Preferably, said polypeptide is as defined previously.

Said medicament can further include at least one immunosuppressive agent for treating and/or preventing inflammatory diseases, inflammatory conditions, or autoimmune diseases.

A eighth aspect of the invention relates to the use of a polypeptide comprising a sequence selected in the group comprising SEQ ID NO.3, its orthologs and derivatives thereof, a polynucleotide coding for said polypeptide or a vector comprising said polynucleotide, for the manufacture of a medicament for treating and/or preventing bacterial, viral, fungal or parasitic infection and/or for treating and/or preventing cancer.

Preferably, said polypeptide is as defined previously.

A ninth aspect of the invention relates to a method for treating and/or preventing preventing inflammatory diseases such as rheumatoid arthritis, atopic dermatitis, psoriasis, multiple sclerosis, diabetes, lupus and inflammatory bowel diseases, inflammatory conditions such as tissue graft or organ rejection, or autoimmune diseases in a subject comprising the step of administrating an effective amount of composition comprising a polypeptide comprising a sequence selected in the group comprising SEQ ID NO.3, its orthologs and derivatives thereof, a polynucleotide coding for said polypeptide or a vector comprising said polynucleotide to said subject.

Preferably, said polypeptide is as defined previously.

According to the present invention, an "effective amount" of a composition is one which is sufficient to achieve a desired biological effect, in this case suppressing or inhibiting cellular immune response. It is understood that the effective dosage will be dependent upon the age, sex, health, and weight of the recipient, kind of concurrent treatment, if any, frequency of treatment, and the nature of the effect desired. The ranges of effective doses provided below are not intended to limit the invention and represent preferred dose ranges. However, the preferred dosage can be tailored to the individual subject, as is understood and determinable by one of skill in the art, without undue experimentation.

A tenth aspect of the invention relates to a method for treating and/or preventing bacterial, viral, fungal or parasitic infection and/or for treating and/or preventing cancer in a subject comprising the step of administrating an effective amount of composition comprising a polypeptide comprising a sequence selected in the group comprising SEQ ID NO.3, its orthologs, and derivatives thereof, a polynucleotide coding for said polypeptide or a vector comprising said polynucleotide to said subject.

Preferably, said polypeptide is as defined previously.

According to the present invention, an "effective amount" of a composition is one which is sufficient to achieve a desired biological effect, in this case at least one of cellular immune response.

In the following, the invention is described in more detail with reference to amino acid sequences, nucleic acid sequences and the examples. Yet, no limitation of the invention is intended by the details of the examples. Rather, the invention pertains to any embodiment which comprises details which are not explicitly mentioned in the examples herein, but which the skilled person finds without undue effort.

### EXAMPLES

### 1) Material and methods

### Cells

PBMC were isolated from heparinized venous blood obtained from healthy donors, by density gradient centrifugation over MSL (PAA LABORATORIES). Fresh PB-NK cells were purified using a magnetic-activated cell sorter (MACS) and a NK cell isolation kit according to the manufacturers' recommendations (MILTENYI BIOTEC). PB-NK cell purity was shown to be >90%. For activation, cells were cultured for 72 h in RPMI 1640 supplemented with 10% heat-inactivated human serum, penicillin (100 IU/ml), streptomycin (100 µg/ml), L-glutamine (2 mM) (INVITROGEN) and IL-15 (10 ng/ml; PEPROTECH). Jurkat cells and derived transfectants, and 221 target cells, were cultured in RPMI 1640 medium supplemented with penicillin/streptomycin, L-glutamine, and 10% heat-inactivated fetal calf serum (FCS; PERBIO SCIENCE).

### Reverse transcription and cDNA amplification (RT-PCR)

Total RNA was isolated using the Trizol reagent according to the manufacturer's instructions (INVITROGEN). RNA (5 µg) was reverse transcribed using 500 ng of an oligo-dT₍₁₂₋₁₈₎ primer (INVITROGEN) and the Powerscript reverse transcriptase (CLONTECH). The specific primers for the amplification of the cDNA corresponding to the GPI-anchored CD160 molecules were 5'-ATGCTGTTGGAACCCGGCAGAG-3' (SEQ ID NO.10; forward) and 5'-TTACAAAGCTTGAAGGGCCAC-3' (SEQ ID NO.11; reverse). The same forward primer in combination with the reverse primer 5'-TCAGTGAAACTGGTTTGAACTTTCCTG-3' (SEQ ID NO.12) were used for the detection of the cDNA coding for the transmembrane isoforms. PCR were performed according to standard procedures, and amplified products were finally separated by electrophoresis on 1% agarose gel.

### Expression vectors and transfection

CD160 and CD160-TM full length cDNA were generated by PCR using the pair of primers corresponding to each type of isoforms, with the exception that a Flag-tag coding sequence was added at the 5' end of the CD160-TM reverse oligonucleotide.

The chimeric CD8-CD160TM construct consists of the extracellular and transmembrane region of CD8a fused to the intracellular part of CD160-TM. It was generated by separately amplifying the cDNA encoding the extracellular and transmembrane domains of CD8a using a CD8a-specific 5' primer (5'-ATGGCCTTACCAGTGACCGCCTTG-3', SEQ ID NO.13) and a chimeric 3' primer (5'-GGGGTGCTTACGGCTCTTTTGGAGTTGCAGTAAAGGGTGATAACCAG-3', SEQ ID NO.14), and the coding sequence corresponding to CD160-TM intracellular domain with an overlapping 5' primer (5'-CTGGTTATCACCCTTTACTGCAACTCCAAAAGAGCCGTAAGCACCCC-3', SEQ ID NO.15) and CD160-TM-specific 3' primer. The two overlapping fragments were used as templates for a PCR with CDBa-5' and CD160-TM-3' primers.

Following in gel purification with the QiaEx II gel purification kit (QIAGEN), each cDNA was ligated into the pEF6 expression vector according to the manufacturer's recommendation (INVITROGEN). The integrity of the inserted sequence was finally assessed by double-strand sequencing.

Jurkat cells were transfected by electroporation with 30 µg of the desired expression vector using a Gene Pulser II (BIORAD) with settings at 250 V and 950 µF. After 48 h of recovery, cells were placed in culture medium containing x µg/ml of blasticidine for selection. The synthesis of CD160 and CD160-TM by the growing clones was assessed by flow cytometry or anti-Flag immunoprecipitation and immunoblot analysis, respectively.

### Generation of anti-CD160-TM specific antibodies

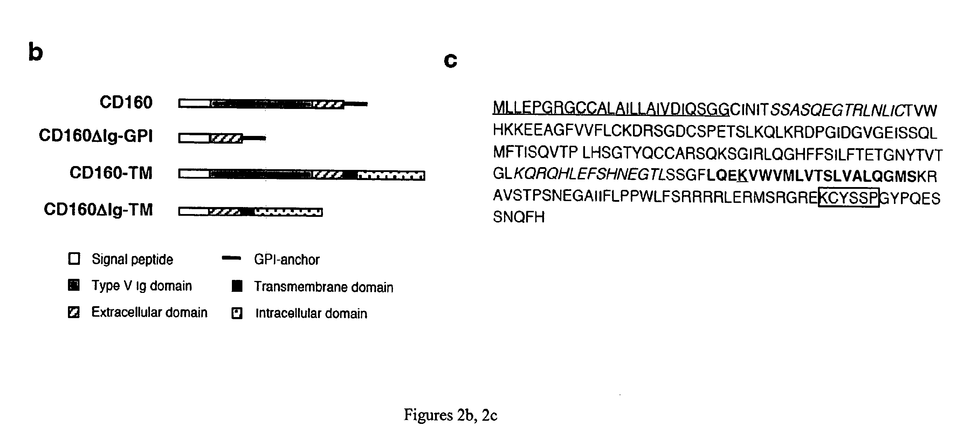

Two rabbits were immunized with peptides located within the extracellular Ig domain (SSASQEGTRLNLIC, aminoacids 31-44, SEQ ID NO.16) and the juxtamembranal region (KQRQHLEFSHNNEGTL, aminoacids 144-158, SEQ ID NO.17) of CD160 transmembrane isoforms (see Fig. 2c). Each rabbit antiserum was further affinity purified on each individual peptide, and the specificity of the purified Ab assessed by immunostaining on CD160-TM-expressing cells.

### Flow cytometry

Cells were incubated with the anti-CD160 mAb BY55 (IgM; 1 µg/test) or affinity purified anti-CD160 polyclonal antibodies (3 µg/test) for 30 min at 4°C. An anti-CD34 mAb (5T-147; IgM) or rabbit pre-immune serum were used as negative controls, respectively. The cells were washed twice in PBS, and further labeled with FITC-conjugated goat anti-mouse or goat anti-rabbit Ig. After extensive washes, cells were analyzed using an EPICS XL apparatus (BECKMAN-COULTER).

### Cell stimulation

Transfected Jurkat cells were incubated with control mouse IgG or anti-CD8 mAb (1µg/ml) and cross-linked with rabbit anti-mouse IgG Ab for 20 min at 37°C. Cells were harvested immediately after stimulation and lysed in 1% NP40 lysis buffer (1% NP40, 20 mM Tris-HCl pH 7.5, 150 mM NaCl, 1mM Na vanadate, 10mM NaF, 1mM phenylmethylsulfonylfluoride, 1µg/ml aprotinin, and 1µg/ml leupeptin). Cellular lysates were analyzed by SDS-8%PAGE and trasferred onto a nitrocellulose membrane.

### Immunoprecipitation and immunoblot analysis

Cells were lysed in 1% NP40 lysis buffer (1% NP40, 20 mM Tris-HCl pH 7.5, 150 mM NaCl, 1mM Na vanadate, 10mM NaF, 1mM phenylmethylsulfonylfluoride, 1µg/ml aprotinin, and 1µg/ml leupeptin) for 1 h at 4°C. Post-nuclear supernatants were subjected to immunoprecipitation using 5 µg of anti-Flag mAb (M2; Sigma-Aldrich) and protein G-sepharose beads. After washes, the precipitated proteins were eluted, separated by SDS-10% PAGE and transferred onto a nitrocellulose membrane. Immunoblot analysis was performed using either the anti-Flag (5 µg/ml) or an anti-phosphotyrosine (4G10, 1 µg/ml; UPSTATE BIOTECHNOLOGIES INC.) mAb. Horseradish peroxidase-conjugated anti-mouse Ig antibodies (JACKSON IMMUNORESEARCH) were used as secondary antibodies. The immunoreactive proteins were visualized by autoradiography using an enhanced chemiluminescence detection system (AMERSHAM BIOSCIENCES). For cells that were stimulated before lysis, membranes were blotted with a specific anti-phospho-Erk1/2 mAb (Sigma-Aldrich) and reprobed with purified polyclonal anti-Erk1/2 antibodies (Cell Signaling Tecnology).

### Proliferation and Cytotoxic assays

The NK cell cytotoxicity was tested using standard procedures. Briefly, the 221 target cells were labeled with Na⁵¹CrO₄, washed and plated in a V-bottomed 96-well plate. The effector cells were then added at various E:T ratios, and the co-culture was processed for 4 h at 37°C. When indicated, rabbit pre-immune serum or purified anti-CD160-TM antibodies (1 µg/well) were added to the reaction medium. Cell supernatant from each well was finally collected, and their radioactive content monitored using a gamma-counter (Packard). All conditions were done in triplicate, and the percentage of specific lysis determined as previously reported (MAIZA *et al.,* 2002, abovementioned).

As detailed elsewhere, Jurkat cells (10⁴/well) were activated with immobilized control mouse IgG1 or anti-CD8 mAb for 6h at 37°C. Cells were then pulsed with 1 mCi of ³H-thymidine for the next 6h, and ³H-thymidine incorporation was measured in a liquid scintillation counter (TopCount; Packard Instrument, Meriden, CA). All conditions were done in triplicate.

### 2) Identification of new CD160 mRNAs

GPI-anchored CD160 molecule has been previously characterized as an activatory receptor able to trigger NK cell lysis following engagement by its physiological ligand HLA-C (LE BOUTEILLER *et al.,* 2002, abovementioned; BARAKONYI *et al.,* 2004, abovementioned). To investigate CD160 functions and potential molecular associations, experiments were conducted to generate a full length cDNA construct for subsequent cloning into an eukaryotic expression vector. Total RNA were isolated from NK92 cells and subjected to reverse transcription. PCR were realized using a pair of primers corresponding to the 5' and 3' ends of CD160. Amplification of β actin cDNA was performed in parallel as internal control.

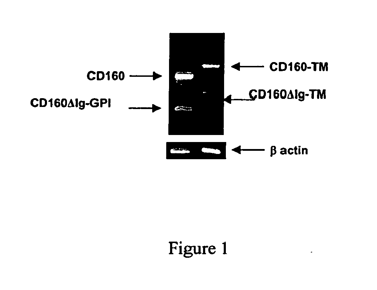

The figure 1 shows the results of the RT PCR experiments.

Unexpectedly, the results established that the RT-PCR experiments performed on total RNA isolated from the NK cell line NK92 constantly led to the detection of two amplified products (CD160 and CD160ΔIg-TM, Fig. 1 lane 1).

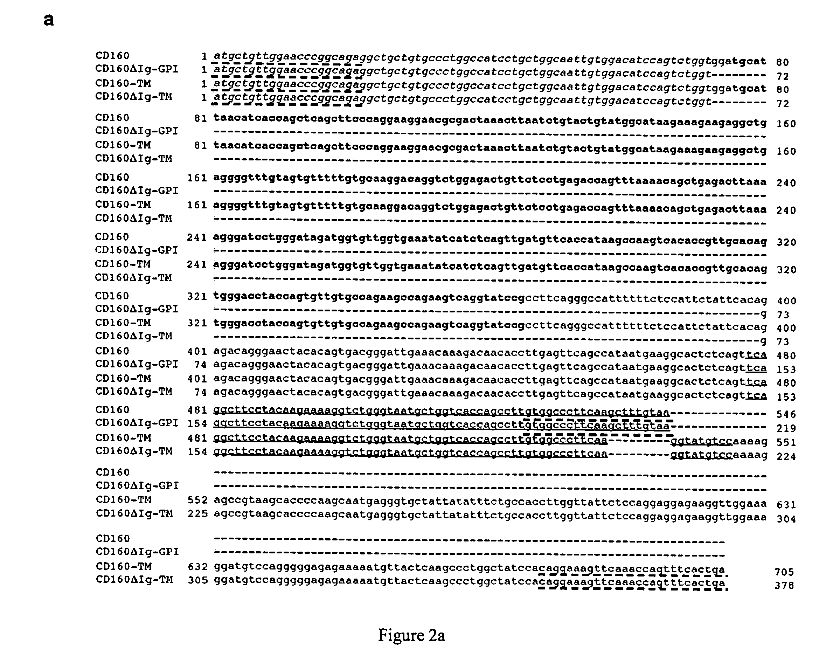

Thus a 550 bp cDNA corresponding to CD160 full coding sequence was observed, as well as a second product of approximately 220 bp. Because additional verifications confirmed the specific amplification of the shorter product, its purification and sequence analysis were undertaken. The results obtained revealed that this cDNA encoded a truncated form of CD160, as it corresponds to a CD160 mRNA (SEQ ID NO.9) that lacks the coding region for the Ig domain (Fig. 2a). This deletion results in a putative GPI-anchored protein devoid of extracellular Ig domain (Fig. 2b). We will refer to this protein to as CD160ΔIg-GPI (SEQ ID NO.2).

We further realized a computer assisted sequence comparison of the newly identified mRNA against available databases.

Following this comparison, the results established that we found a third mRNA presenting a high level of identity with the CD160ΔIg-GPI transcript (GeneBank accession number AK128370, SEQ ID NO.18, Fig. 2a). Upon translation, this third transcript would lead to the synthesis of a CD160 protein presenting no extracellular Ig domain, but exhibiting a transmembrane and an intracellular domain (Fig. 2b). This putative variant of CD160 was therefore called CD160ΔIg-TM (SEQ ID NO.19).

To assess if this third mRNA was synthesized by the NK92 cell line, specific primers allowing the amplification of CD160ΔIg-TM cDNA were designed (see Fig. 2a). As previously observed for the synthesis of CD160 cDNA, two specific products of roughly 400 and 700 bp were detected (Fig. 1, lane 2). The sequencing of these two PCR products showed that the smaller one corresponded to CD160ΔIg-TM cDNA, while the larger one coded for a fourth possible isoform of CD160. Indeed, this cDNA presents an open reading frame coding for the complete extracellular domain of CD160, but then enters into a nucleotide sequence coding for a transmembrane and an intracellular domain (SEQ ID NO.9, Fig. 2a). The corresponding polypeptide would therefore be a transmembrane protein sharing the same extracellular domain that the original GPI-anchored molecule (Fig. 2b). We will refer to this isoform to as CD160-TM (SEQ ID NO.1).

The figure 2 shows the cDNA sequences and molecular organization of CD160 isoforms. (a) Sequence comparison of CD160 isoforms cDNA. The predicted coding region for the signal peptide (italic), the Ig-like domain (bold) and the hydrophobic region (underlined) are delineated. The sequence of the primers used for cDNA amplification is also delineated (dashed line). (b) Schematic representation of the predicted molecular organization of CD160 isoforms. Two types of proteins can be distinguished on the criteria of their mode of insertion into the membrane (GPI or TM), or of the presence or absence of the Ig-like domain within their extra cellular part. The putative polypeptides share the same signal peptide as well as a X amino acid sequence in their extra cellular moiety. (c) Predicted amino acid sequence of CD160-TM protein. The amino acids predicted to form the signal sequence are underlined. The hydrophobic portion, deduced from a Chou and Fasman hydrophobicity analysis, and which contains a positively charged Lys (K) residue, is in bold. The intracellular KCYSSP motif, similar to the one found in NKp80 sequence, and where is located a putative tyrosine phosphorylation site, is boxed.

Thus, we demonstrated that four CD160 transcripts were synthesized by NK92 cells, two coding for GPI-anchored molecules, and two corresponding to their transmembrane counterparts. In addition, each pair of GPI- and transmembrane proteins can be distinguish from the other according to the presence or absence of the Ig domain within their extracellular moiety. Complementary sequence comparisons of the four cDNA sequences with genomic DNA showed that all transcripts were produced by alternative splicing of *CD160* gene.

### 3) Expression of CD160 transcripts in peripheral blood NK cells

To further investigate the physiological relevance of the identification of four CD160 transcripts, and thus of the potential synthesis of four CD160 isoforms, RT-PCR experiments were realized on freshly isolated peripheral blood (PB) NK cells. PB-NK cells were purified from the peripheral blood of a healthy donor, and cultured in the presence (+) or absence (-) of IL-15 for 72 h.

Total RNA were extracted from purified PB-NK cells, reverse-transcribed and amplified with the primer pair specific for either the GPI-anchored or the transmembrane molecules (see Fig. 2a). β actin cDNA synthesis was used as internal control.

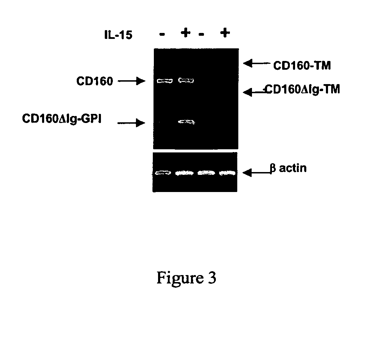

The figure 3 shows the results of CD160 isoforms mRNA synthesis upon IL-15 activation of PB-NK cells. The position of the cDNA corresponding to each isoform is as indicated.

As shown in Fig. 3, the transcripts corresponding to the two GPI-bound proteins (CD160 and CD160ΔIg-GPI) were present in resting PB-NK cells, while the one encoding the transmembrane isoforms remained undetectable (Fig. 3). Interestingly we found that the incubation of the cells with IL-15 resulted in the neo-synthesis of CD160-TM and CD160ΔIg-TM mRNA (Fig. 3, right panel). In contrast, the transcription of the GPI-isoforms mRNA did not seem to be modified by the activation process (Fig. 3, left panel).

Thus, unlike the NK92 cell line that constitutively expressed the four CD160 transcripts (Fig. 1), PB-NK cells present an activation-dependent synthesis of CD160 mRNAs, as the potential expression of the transmembrane molecules can only be achieved following activation.

### 4) The synthesis of CD160ΔIg-TM and CD160-TM transcript is restricted to activated NK cells

To further evaluate the cellular specificity of CD160 TM-isoforms transcripts expression, RT-PCR was conducted on total RNA extracted from PB-sorted cells, tissue-isolated cells, or from various established cell lines. The results are presented in table I as follows.

**Table I**

| Samples | | GPI | TM |
|---|---|---|---|
| Fresh tissues | PBMCs | + | - |
| | Activated PBMCs | + | + |
| | Cord blood | + | - |
| | CD34⁺ cells | - | - |
| | Thymocytes | - | - |
| | NK cells | + | - |
| | IL-15-activated NK cells | + | + |
| | CD4⁺ T cells | - | - |
| | IL-15-activated CD4⁺ T cells | + | - |
| | CD8⁺ T cells | + | - |
| | IL-15-activated CD8⁺ T cells | + | - |
| Lymphoid cell lines | Thymic clone (B20) | - | - |
| | T cell (LSO) | + | - |
| | CD4⁺ T cell (JAF43) | - | - |
| | CD8⁺ T cell (JF1) | + | - |
| Tumoral cell lines | B cell (Daudi) | - | - |
| | T cell (Jurkat, Molt4) | - | - |
| | NK cell (NK92, YT indi, NKL) | + | + |

The results reported in Table I show that among the PB cell types tested, the transcripts encoding CD160 TM-isoforms were only detected in NK cells following IL-15 treatment. These mRNA were also not found in thymocytes, cord blood cells or CD34⁺ cells. In agreement with previous studies, the GPI-isoforms transcripts showed a broader distribution pattern as they were detected in resting and activated PB-NK and - CD8⁺ lymphocytes (ANUMANTHAN *et al.,* 1998, abovementioned; MAIZA *et al.,* 1993, abovementioned), and in activated PB-CD4⁺ T cells (ABECASSIS et al., J. Invest. Dermatol., vol.187, p:2065-2072, 1998). Additionally, CD160ΔIg-TM and CD160-TM transcripts were successfully amplified from NK tumoral cell lines but not from established T or B cell lines (Table I). These data strongly suggested that CD160 TM-isoforms are exclusively expressed by activated NK cells and by their transformed counterparts.

### 5) Switch in CD160 isoform expression upon activation of PB-NK cells

We next wanted to establish if the activation-dependent synthesis of the mRNA corresponding to the transmembrane isoforms was correlated to the expression of the proteins within the cells. To this aim, polyclonal antibodies directed towards the transmembrane isoforms were generated by the immunization of rabbits with a mix of two peptides specific for CD160 molecules, and located within the Ig-like and the juxtamembranal domain of the protein (see the Material and Methods section, and Fig. 2c, for sequence details).

Each serum was further affinity purified on each individual peptide, and the reactivity and specificity of the purified anti-CD160-TM antibodies assessed on Jurkat cells transiently transfected with an expression vector coding for either CD160 or CD160-TM molecule. The anti-CD160 mAb BY55 was used as a control and rabbit pre-immune serum, or isotype-matched anti-CD34 mAb, were used for negative controls. Following addition of the appropriated FITC-coupled secondary antibodies, cells were analyzed by flow cytometry.

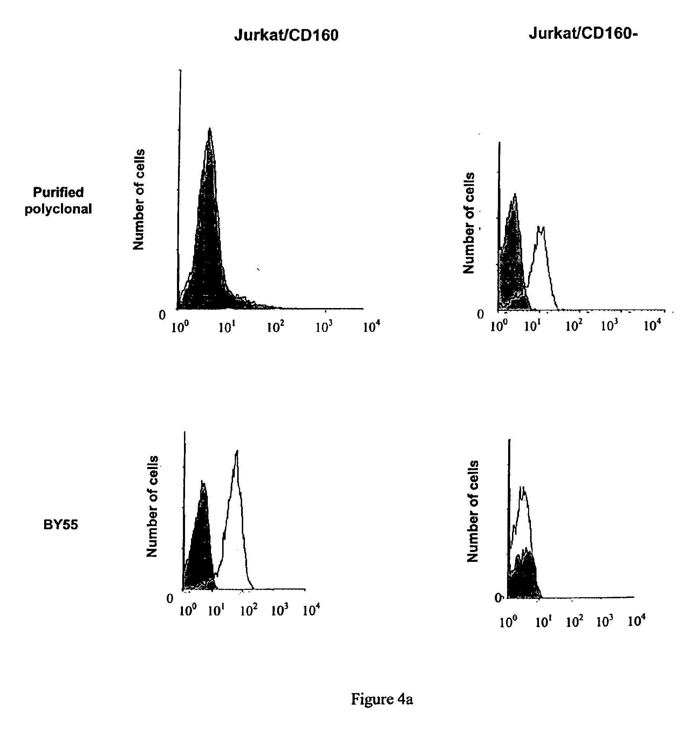

The figure 4a shows the characterization of anti-CD160-TM polyclonal antibodies (upper panel) or the results obtained with the anti-CD160 mAb BY55 (lower panel).

The results obtained clearly demonstrated that one of the serum, directed against the Ig-like domain peptide, has no reactivity towards CD160-transfected cells, but efficiently labeled the CD160-TM transfectants (Fig. 4a, upper panel). Conversely, the anti-CD160 mAb BY55 positively stained the CD160-expressing cells but gave no signal on CD160-TM-transfected cells (Fig. 4a, lower panel). Regarding this latter observation, one should mention that a weak reactivity was obtained with BY55 mAb when CD160-TM cells were subjected to a fixation step prior to immunolabeling (data not shown). Thus, we obtained purified polyclonal antibodies that, according to their peptide recognition ability, allow the detection of CD160-TM molecule but not of its ΔIg counterpart.

The obtention of antibodies specific for CD160-TM isoform further allowed us to investigate the expression pattern of the GPI-anchored molecules *versus* the full length transmembrane protein on PB-NK cells. Freshly isolated PB-NK cells were grown in medium alone (-IL-15) or supplemented with IL-15 (+ IL-15) and immunolabeling of resting or IL-15-activated PB-NK cells were performed using either BY55 mAb or the anti-CD160-TM polyclonal antibodies.

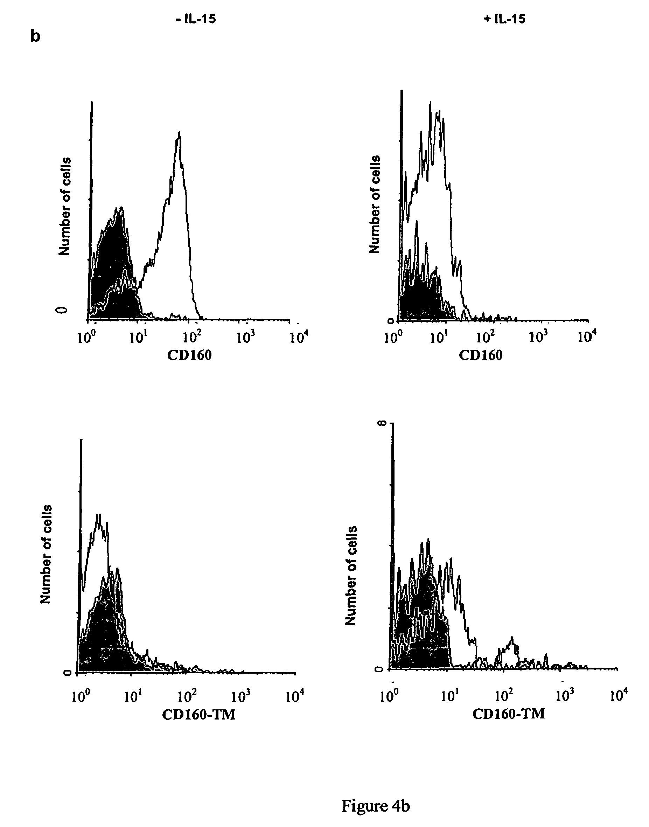

The figure 4b shows the results obtained with the anti-CD160 antibody (upper panel) or anti-CD160-TM antibody (lower panel).

As previously observed, CD160 is present on control cells, and becomes undetectable upon activation (Fig. 4b, upper panel). We established that this loss in GPI-anchored CD160 detection resulted from an activation-dependent proteolytic cleavage of the molecule, leading to the release of a soluble form (GIUSTINIANI et al. (J. Immunol., vol.178(3), p:1293-1300, 2007). Moreover, in agreement with CD160 mRNA analysis, no CD160-TM is expressed by resting PB-NK cells, but its cell surface expression is induced following IL-15-treatment (Fig. 4b, lower panel). Thus it seems that a switch in CD160 isoforms expression occurs at the surface of PB-NK cells, the GPI-molecules being replaced by the transmembrane protein following activation.

### 6) Molecular characterization of CD160-TM

Our observation that the clearing of GPI-anchored CD160 from the NK cell surface parallels the appearance of the transmembrane molecules prompted us to further investigate the structure and functions of CD160-TM. Jurkat cells were transfected with an empty control vector (control) or with a construction coding for a Flag-tagged CD160-TM fusion protein (CD160-TM-Flag coding construct).

After lysis, cellular extracts were subjected to immunoprecipitation using an anti-Flag mAb, and the immune complexes were separated by SDS-PAGE. Immunoblot analysis was then conducted with the anti-Flag mAb followed by a HRP-conjugated anti-mouse Ig.

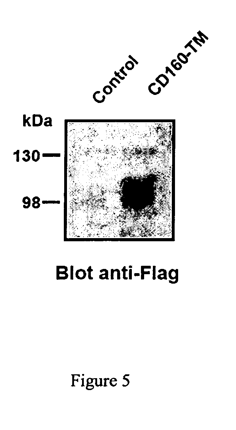

The figure 5 shows the results for the detection of CD160-TM showing that CD160-TM corresponds to a 100 kDa protein.

Thus, CD160-TM is expressed as a homotrimeric molecule, which appears to be unsensitive to reducing agent, as observed for the original CD160 (ANUMANTHAN *et al.,* 1998, abovementioned).

### 7) CD160-TM triggering enhances the NK lymphocyte cytotoxicity

To determine whether CD160-TM might trigger activating or inhibitory signals following its engagement, the cytotoxic potential of IL-15-activated PB-NK cells was evaluated by performing cytotoxic assays. Freshly isolated PB-NK lymphocytes were activated with IL-15 and further tested for their ability to lyse 221 target cells.

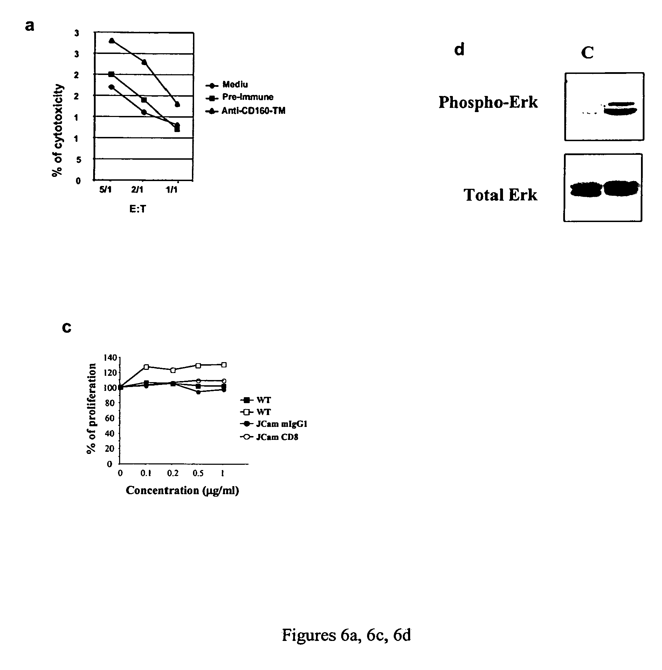

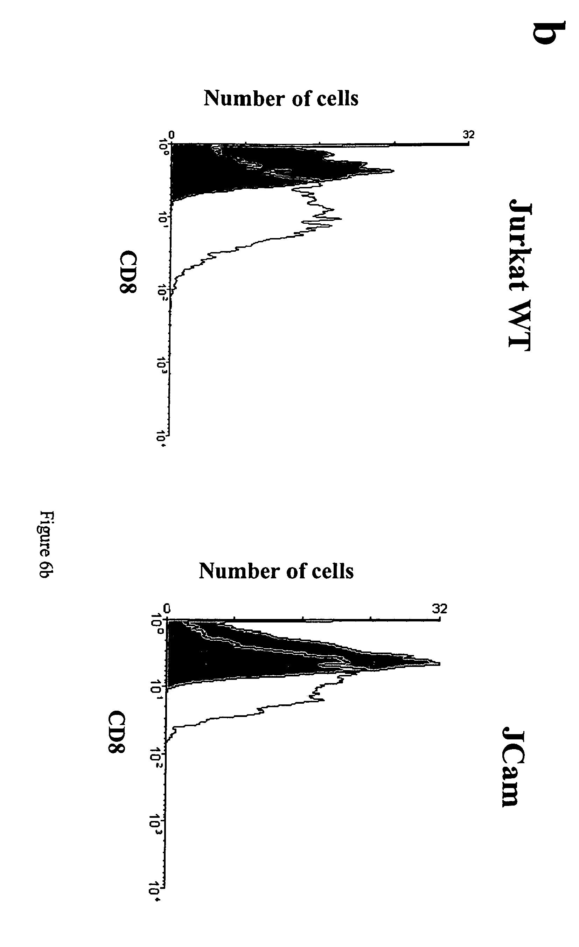

The Figure 6 shows (a) the cytotoxic activity of IL-15 activated PB-NK cells against 221 target cells measured in the absence of antibodies (◆), or in the presence of rabbit pre-immune serum (■) or purified anti-CD160-TM polyclonal antibodies (Δ). Results from a representative experiment are shown and presented as mean of triplicates ± SD.

The results show that IL-15-activated NK cells efficiently recognize the target cells, as demonstrated by the detection of cytotoxic activity (see figure 6a). In addition, the results established that the addition of anti-CD160-TM antibodies to the PB-NK cells, prior to contact with the target cells, resulted in an enhancement of their cytolytic potential. Indeed, a 30-40% increase of the specific lysis was detected under these conditions, while no effect was noted when the corresponding rabbit pre-immune serum was used for the pre-incubation step (see figure 6a). Thus, it seems that CD160-TM triggering at the NK cell surface leads to the generation of activating signals, and consequently to an enhanced cytotoxic activity.

### 8) CD160-TM intracellular domain is sufficient to induce a cellular proliferation and to activate Erk pathway

The molecular basis underlying CD160-TM activating function was next analyzed. The protein sequence analysis of CD160-TM revealed the presence, within its transmembrane domain, of a positively charged lysine residue (see Fig. 2c). An association of the molecule with ITAM-bearing adaptors through the establishment of a stable salt bridge was therefore considered. However, by co-expressing CD160-TM together with DAP10, DAP12, ζ or FcεRIγ in COS cells, we did not evidence any association between these adaptor proteins and CD160-TM (data not shown). Another feature of CD160-TM is the presence, in its intracellular domain, of two tyrosine residues at positions 220 and 225, which might represent potential docking sites for signaling molecules upon phosphorylation. To investigate if CD160-TM function was dependent on its intracellular domain, a chimeric construct coding for the CD8α extracellular and transmembrane domains fused to CD160-TM cytoplasmic portion was generated.

Wild type (WT) or Lck-deficient (JCam) Jurkat cells stably expressing the chimeric protein were selected (see Fig. 6b for expression) and the activating potential of the chimera tested in proliferation assays.

The figure 6(b) shows the expression of CD8-CD160TM molecule in wild type (WT) or Lck-deficient (JCam) Jurkat cells after a stable transfection with a CD8-CD160TM chimera. Expression of the chimeric protein was assessed by immunolabeling using a PE-conjugated anti-CD8 mAb vs. an isotype-matched control Ig.

The figure 6(c) shows the results of an activation of WT or Lck-deficient CD8-CD160TM transfectants with increasing concentrations of either mouse IgG1 (mIgG1) or anti-CD8 mAb, as indicated. Results were expressed as the percentage of proliferation, with 100% corresponding to basal cell growth.

We observed, that while the transfected WT cells did not proliferate in response to control mouse IgG1, a substantial enhancement of their growth rate, corresponding to a 20-30% increase of their proliferation level, was obtained following CD8 triggering (see figure 6c). Importantly, the CD8-induced proliferation was no longer detected upon triggering of the Lck-deficient JCam transfectants (see figure 6c). In addition, the CD8-mediated proliferation of the WT cells was completely abolished in the presence of the Src-family kinases inhibitor PP2 (data not shown).

To further characterize the signaling pathway engaged upon CD160 triggering, Jurkat cells stably expressing the CD8-CD160-TM chimera were either left unstimulated or activated with an anti-CD8 mAb. The corresponding cellular lysates were then analyzed for the activation of Erk by Western blot using an anti-phospho-Erk mAb.

The figure 6(d) shows the results of an incubation of WT Jurkat cells expressing the CD8-CD160TM chimera with mouse IgG1 (C) or activated with an anti-CD8 mAb. Immunoblot analysis was performed on total cell lysates using an anti-phospho-Erk1/2 mAb. Equal protein loading was assessed by reprobing the membrane with anti-Erk1/2 antibodies.

We observed that the triggering of the CD8-CD160-TM chimera efficiently recruited Erk signaling cascade, as assessed by the detection of a significant Erk phosphorylation. Altogether the data presented in figure 6 established that CD160-TM represents an activating receptor, which function is dependent on p56^{1ck} kinase activity, and that its recruitment led to the activation of Erk signaling pathway.

### 9) CD160ΔIg-GPI

To investigate the functional role of CD160ΔIg-GPI, a cDNA encoding a C-terminal Flag (DYKDDDK)-Tagged soluble CD160ΔIg-GPI (sCD160ΔIg-GPI-Flag, SEQ ID NO.20) is generated by PCR amplification of the sequence corresponding to amino-acids 1-51 of CD160ΔIg-GPI. After purification, the resulting PCR product is ligated into the pcDNA3 expression vector (INVITROGEN), and the construct double-strand is sequenced.

Simultaneously, a cDNA encoding a C-terminal Flag (DYKDDDK)-Tagged soluble CD160 (sCD160-Flag, SEQ ID NO.21) is generated by PCR amplification of the sequence corresponding to amino-acids 1-160 of CD160 as described in GIUSTINIANI *et al.* (2007, abovementioned).

COS7 cells are transiently transfected with with the pcDNA vector, the sCD160-Flag expression vector or the sCD160ΔIg-GPI-Flag expression vector using the DEAE dextran method, and subsequently cultured for 72 h in serum free RPMI 1640 medium supplemented with L-glutamine and antibiotics. The produced sCD160-Flag and sCD160ΔIg-GPI-Flag are detected by ELISA with an anti-Flag mAb according to the protocol described in GIUSTINIANI *et al.* (2007, abovementioned).

The sCD160-Flag and sCD160ΔIg-GPI-Flag are purified by immunoprecipitation from transfected COS7 culture medium using CL1-R2 mAb or anti-CD160 ΔIg antibody coupled to protein G-Sepharose beads (AMERSHAM BIOSCIENCES) and eluted in 2mM glycine-HCl ph 2.8. After neutralization, a second immunoprecipitation step is performed with agarose-coupled anti-Flag mAb (SIGMA). sCD160-Flag and sCD160ΔIg-GPI-Flag are finally eluted in 2mM glycine-HCl pH 2.8.. The eluates are neutralized, submitted to dialysis in PBS, and concentrated with CENTRICON (MILLIPORE). The protein concentration in eluates is then estimated on a silver-stained gel by comparison with known quantities of BSA.

In order to determine an eventual interaction between CD160ΔIg-GPI and MHC-class I molecules, a sCD160ΔIg-GPI-Flag binding assay on HLA-Cw3-expressing 721.221 cells is performed, with sCD160-Flag as a control and as described in GIUSTINIANI *et al.* (2007, abovementioned).

The effect of sCD160ΔIg-GPI-Flag on MHC- class I-restricted cytotoxic T lymphocyte (CTL) activities is determined. Therefore, the cytolytic activity of the HLA-A11-restricted human cytotoxic T cell clone JF1 (DAVID et al., J. Immunol., vol.138, p:2831-2836, 1987) is tested against the specific HLA-A11 EBV-transformed B cell line. The target cells are preincubated with a culture supernatant obtained from COS7 cells transfected with either the empty expression vector, the sCD160-Flag coding construct, the sCD160ΔIg-GPI-Flag coding construct, or the sCD160-Flag and sCD160ΔIg-GPI-Flag coding constructs simultaneously.

## Claims

1. An isolated polypeptide comprising a sequence selected in the group comprising SEQ ID NO.1, SEQ ID NO.3, their orthogs, and derivatives thereof.

2. The isolated polypeptide of claim 1, wherein said polypeptide comprises a sequence selected in the group consisting of SEQ ID NO.1, its orthologs, and derivatives thereof.

3. The isolated polypeptide of claim 1, wherein said polypeptide comprises a sequence selected in the group consisting of SEQ ID NO.3, its orthologs, and derivatives thereof.

4. The isolated polypeptide of claim 3, wherein said polypeptide comprises a signal peptide enabling the secretion of the polypeptide, preferably wherein said polypeptide comprises the sequence SEQ ID NO.4.

5. The isolated polypeptide of claim 3, wherein said polypeptide comprises a Glycosyl Phosphatidyl Inositol (GPI) anchor domain, preferably wherein said polypeptide comprises the sequence SEQ ID NO.5

6. The isolated polypeptide of claim 3, wherein said polypeptide does not comprise any Ig domain, preferably wherein said polypeptide does not comprise the sequence SEQ ID NO.6

7. The isolated polypeptide of claim 3, wherein said polypeptide does not comprise any transmembrane domain, preferably wherein said polypeptide does not comprise the sequence SEQ ID NO.7.

8. The isolated polypeptide of claim 3, wherein Advantageously, said polypeptide comprises a sequence selected in the group consisting of SEQ ID NO.2, its orthologs and derivatives thereof.

9. A polynucleotide comprising a nucleic acid sequence encoding for a polypeptide as defined in any one of claims 1 to 8.

10. The polynucleotide of claim 7, wherein said polynucleotide comprises a sequence selected in the group comprising SEQ ID NO:8 and SEQ ID NO:9.

11. A vector comprising the polynucleotide as defined in claim 9 or 10.

12. A host cell genetically engineered with the polynucleotide as defined in claim 9 or 10, or with the vector defined in claim 11.

13. A pharmaceutical composition comprising a polypeptide as defined in any one of claims 3 to 8, a polynucleotide coding for said polypeptide or a vector comprising said polynucleotide, and optionally a pharmaceutically acceptable carrier.

14. A method for screening for antagonists and/or agonists of the polypeptide as defined in claim 2 comprising:
a. expressing the receptor corresponding to the polypeptide as defined in claim 2 on the surface of a cell;
b. contacting the cell with a receptor ligand and a compound to be screened;
c. determining whether a second signal is generated from the interaction of the ligand and the receptor; and
d. identifying if the compound to be screened is an agonist or an antagonist.

15. A use of a polypeptide as defined in any one of claims 3 to 8, a polynucleotide coding for said polypeptide or a vector comprising said polynucleotide, for the manufacture of a medicament for treating and/or preventing inflammatory diseases such as rheumatoid arthritis, atopic dermatitis, psoriasis, multiple sclerosis, diabetes, lupus and inflammatory bowel diseases, inflammatory conditions such as tissue graft or organ rejection, or autoimmune diseases in a subject.

16. A use of a polypeptide as defined in any one of claims 3 to 8, a polynucleotide coding for said polypeptide or a vector comprising said polynucleotide, for the manufacture of a medicament for treating and/or preventing bacterial, viral, fungal or parasitic infection and/or for treating and/or preventing cancer.
